(19) Europäisches Patentamt
European Patent Office
Office européen des brevets

(11) **EP 2 878 268 B1**

## (12) EUROPEAN PATENT SPECIFICATION

(45) Date of publication and mention
of the grant of the patent:
**27.05.2020 Bulletin 2020/22**

(51) Int Cl.:
*A61B 6/00* *(2006.01)*   *A61B 6/14* *(2006.01)*
*A61B 6/04* *(2006.01)*   *A61B 6/06* *(2006.01)*
*G06T 7/00* *(2017.01)*

(21) Application number: **14195204.4**

(22) Date of filing: **27.11.2014**

(54) **Method and apparatus time adjusting technical exposure factors during a radiographic acquisition**

Verfahren und Vorrichtung zur Einstellung technischen Belichtungsfaktoren während einer radiografischen Erfassung

Procédé et appareil d'ajustement des facteurs d'exposition pendant une acquisition radiographique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2013 IT BO20130670**

(43) Date of publication of application:
**03.06.2015 Bulletin 2015/23**

(73) Proprietor: **CEFLA SOCIETA' COOPERATIVA**
**40026 Imola (BO) (IT)**

(72) Inventors:
- **Manuzzato, Gianluca**
  **40026 Imola (BO) (IT)**
- **Bruno, Andrea**
  **40026 Imola (BO) (IT)**

(74) Representative: **Karaghiosoff, Giorgio Alessandro**
**c/o Praxi Intellectual Property S.p.A. - Savona**
**Via F. Baracca 1R, 4° piano**
**"Il Gabbiano"**
**17100 Savona (IT)**

(56) References cited:
**EP-A2- 2 574 280      DE-A1-102010 042 761**
**US-A- 5 124 913       US-A- 5 995 583**
**US-A1- 2007 058 772**

- **ELBAKRI IDRIS ET AL: "Automatic exposure control for a slot scanning full field digital mammography system", MEDICAL PHYSICS, AIP, MELVILLE, NY, US, vol. 32, no. 9, 22 August 2005 (2005-08-22), pages 2763-2770, XP012075447, ISSN: 0094-2405, DOI: 10.1118/1.1999107**

**Description**

The present invention relates to the technical field of dental digital radiographic apparatuses known as panoramic apparatuses. In particular, the invention relates to a method and an apparatus patient specific, real time automatically adjusting the X-ray dose administered to a patient, so as to dispense the minimum dose necessary to get a radiographic image of good quality.

These apparatuses produce a panoramic image of the dental arches of a patient, exposing a patient skull to X-rays. Panoramic radiography (also known as orthopantomography) produces a radiographic image of a curved plan, also known as Welander curve, approximating patient jaws, with blurring of the anatomical structures laying outside a narrow layer around the predesigned curved plane. The set of movements that mechanical parts have to perform to get this result is called trajectory. This technology has been known since the '50s ; for the first 30-40 years (1950-1990) a film used to be exposed to X-rays. Nowadays such apparatuses use digital sensors, converting the X-ray impinging on the X-ray detector into an electric signal, which suitably processed forms a digital image.

The X-ray bundle undergoes attenuation while passing through different patient's tissues; this attenuation determines the degree of exposure of radiographic image. Different tissues attenuate X-rays in different ways according to their density, and this allows to distinguish a tissue from the other. The correct exposure is obtained balancing the intensity of X-ray bundle, so that the tissues of clinical interest are well visible and detailed. In an excessive exposure, low-medium density tissues are crossed by X-rays without an attenuation such as to be detected (overexposure), while too low an exposure entails an excessive X-ray attenuation by more dense tissues, so that they cannot be distinguished from the nearby tissues (underexposure).

In case of overexposure or underexposure a retake may be necessary. One has always to keep into account that X-rays can lead to patient biological damage, as X-rays are ionizing radiations which can damage cell DNA. Therefore, the need to administer the lowest X-ray dose for the single patient under examination is apparent.

Typically, a panoramic apparatus comprises an X-ray source and detector fixed to the ends of a rigid support, turning around the patient. It also comprises a patient positioning and immobilizing device, to hold the patient during the acquisition.

The typical technical e influencing the intensity of the X-rays emitted from the source are:

- X-ray tube power (kV),
- X-ray tube current (mA),
- duration of exposure (sec) and
- film speed.

Originally, in film analog apparatuses, this last parameter used to determine how long a portion of the film was exposed to radiation, and film direction. This parameter in many of today's digital apparatuses was maintained as term, and corresponds to the speed and reading direction of X-ray sensor; elsewhere it is replaced by a parameter called clock TDI. In both cases, its effect on the quality of image exposure is similar.

The exposure for a patient can be adjusted by a professional operator, who can evaluate in advance the right X-ray dose needed for a good radiographic image, on the basis of his/her professional competence. Many manufacturers propose to operator pre-defined settings of these parameters, suitably set according to patient's gender and size, e.g. distinguishing among child, woman, man, and small, medium large size; such a system is described e.g. in US4618974 Siemens. Anyway, the selection of these settings is always left to human operator.

A more recent example is disclosed in document EP 2574280. This document describes a device, a method, and a program for assisting in initial setting of imaging condition, and radiation imaging apparatus and radiation imaging system. In this case, sample data is stored in a console of an electronic cassette (image detection panel). The sample data corresponds to a model of the electronic cassette and includes sample images captured using different doses before a shipment of the electronic cassette. In initial setting processing of an imaging condition including the dose, the sample data is read out and a dose selection screen in which the sample images are arranged is displayed on a display. Similarly to US4618974 the determination of the exposure is made using pre-defined samples and settings and not by considering the patient specific anatomy.

Over the years, automatic adjusting devices were developed, which set the exposure according to a suitably chosen criterion (best contrast, lowest dose, etc.). These devices are known as AEC (Automatic Exposure Control) devices. The solutions nowadays available on the market can be subdivided into two categories:

- parameters adjustment according to a pre-acquisition exposure (also known as scout);
- continuous parameters adjustment during the acquisition (also known as run-time AEC).

A first way of the known art to solve the present technical problem consists in devices based on measuring, through dosimeters or similar devices, X-ray attenuation during scout acquisition. These detectors can be placed in one or more areas on the detector side (both a sensor or a film), and can be fixed, mobile or removable. Such technique is described e.g. in patents US4813060 Siemens, US5425065 Instrumentarium and EP0574368 Orion Yhtymae Oy. The detectors provide a direct measurement of X-ray attenuation through patient's tissues.

The disadvantages of this kind of solution are due to the need of specific mechanisms for dosimeter reading, their maintenance, the managing of their volume and the optimization of a work flow, wherein the attenuation measuring step and the following exposure to obtain images need supports, interfaces and data processing different from each other. When digital detectors replaced films, digital detectors replaced the use of specific dose detectors, since digital detectors can be used both for attenuation measuring during pre-acquisition and for image formation during the acquisition. The use of the same type of detector removes the need of correlating different signals (provided from a dosimeter and a digital detector) to exposure, preventing the need of cross-calibrations and cross-checks between the two systems.

Many of these mechanisms, though, do not take into account patient's structure and anatomical peculiarities, or the human operator must choose suitable exposure parameters according to his/her professional experience and competence, looking at scout image. In this case the time needed for the acquisition becomes longer, and a wide scout or more than one scout are required to localise the anatomical portion of interest. Moreover, the use of a scout requires the administration of a further (small) X-ray dose to patient.

If scout image is too small, or parameter computing algorithm is not specific for the acquired anatomic portion, or operator's intervention is not required in choosing parameters, the automatic setting of emission can become very sensitive to patient's positioning or peculiar bone structure.

Document US 5,124,913 discloses a method for automatically determining the image read out condition in storage phosphor radiography systems. In this document, use is made of a pre scan of the phosphor storage screen. Results of the pre scan are evaluated using a grey scale histogram and the evaluation is used for setting the parameters of the read out gain in the final scan step of the storage phosphors screen. The results of this methods are not directed to the determination of the exposure and thus of the dose of a patient in an x-ray imaging session.

A second way of known art for solving the present technical problem is a continuous time adjustment of exposure during the acquisition; the most popular methods require complex feed-back control mechanisms of X-ray tube. Such a technique is described e.g. in patent US4333012 Morita. According to this solution, a control system must be designed, which, during the acquisition, reads at time t the content of detector, processes it to get suitable emission parameters and adjusts the emission at time t+1 according to them. This entails designing X-ray tubes modulating their emission in very short time, with a very stable answer to input parameters and very short transient state, and a very fast system of detector reading and analysis.

In addition to technical complexity and high cost of this solution, one of its main disadvantages is that in situations of peculiar irregularity of patient's anatomy, or in the presence of metallic prostheses, or of a material much denser than adjoining tissues, the value of parameters computed at time t can be not suitable at the following time.

Using this technique, the image obtained risks to become a puzzle of portions of tissue exposed in very different way during the same acquisition, making comparative analyses very difficult between tissues having similar features, but positioned in points far away from each other, as e.g. in the case of bone density between right and left mandibular condyle.

A third way of solving the present technical problem consists in devices for measuring patient's dimensions of skull and size (e.g. height and skull diameter) based on an alleged correlation between these external measures and bone tissues thickness and density. One method of this kind is described in patent EP1161122 Palodex. Such correlation is purely statistical and does not take into any account the anatomical, pathological or personal peculiarities of the patient (e.g. osteoporosis, bone age, etc.).

Aim of the present invention is solving the technical problem consisting in the adjustment of technical exposure parameters with an automatic adjusting method, free of the above-explained problems, capable of leading to a diagnostically valid panoramic image.

Particularly the adjustment of the exposure shall be determined on the anatomy of each one of the patients being examined and carried out during the imaging session.

This object is achieved by a method and an apparatus having the features of the independent claims. Advantageous embodiments and refinements are specified in claims dependent thereon.

The proposed mechanism is based on the acquisition of a pre-acquisition scout of a well defined patient's anatomic portion, with a simple algorithm for computing the optimal exposure for a given patient.

The present invention has the advantage that, without a specific hardware structure, the attenuation measures performed on the scout provide reliable values of X-rays attenuation due to patient's tissues, and are directly correlated to the exposure degree of the image obtained with the following acquisition.

Another advantage is that the present invention does not require parameters modulation during the acquisition, ensuring the constancy of the exposure for the whole duration of the acquisition and its repeatability.

Further advantages are that the present system is operator-independent, does not require additional elements with respect to the components needed to obtain the image, is of easy realization without the need of control and process devices with respect to those needed for standard use.

Further advantages and properties of the present invention are disclosed in the following description, in which exemplary embodiments of the present invention are explained in detail based on the drawings:

Figure 1 Exemplary panoramic radiographic apparatus;
Figure 2 Histogram of grey levels of a typical panoramic radiographic image;
Figure 3 Histogram of grey levels of a radiographic image to perform the method of the present invention;
Figure 4 Flow diagram of the known art;
Figure 5 Flow diagram of the present invention;
Figure 6 Anatomic area for performing a scout acquisition.

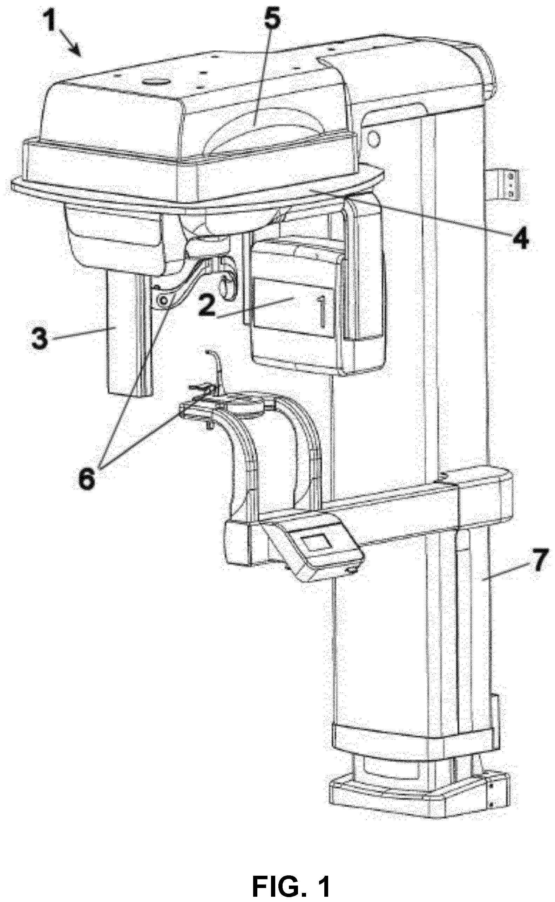

Figure 1 shows a typical panoramic radiographic apparatus, comprising an X-ray source 2 projecting a collimated X-ray bundle across a (not shown) patient, a bi-dimensional X-ray detector 3 positioned so as to measure the intensity of radiation after it crossed the patient, a C-arm 4 on which said X-ray source 2 and detector 3 are fixed on opposed ends, a mechanical system 5 rotating and translating said support around the patient, so as to acquire radiographic images from different positions; a (not shown) electronic system to control and synchronize the working of the various apparatus components. Panoramic apparatus 1 comprises moreover a device 6 for positioning the patient, in Figure 1 consisting of a bite and supports for positioning patient's skull. The position of C-arm 4 can be adjusted to patient's height thanks to vertically mobile post 7.

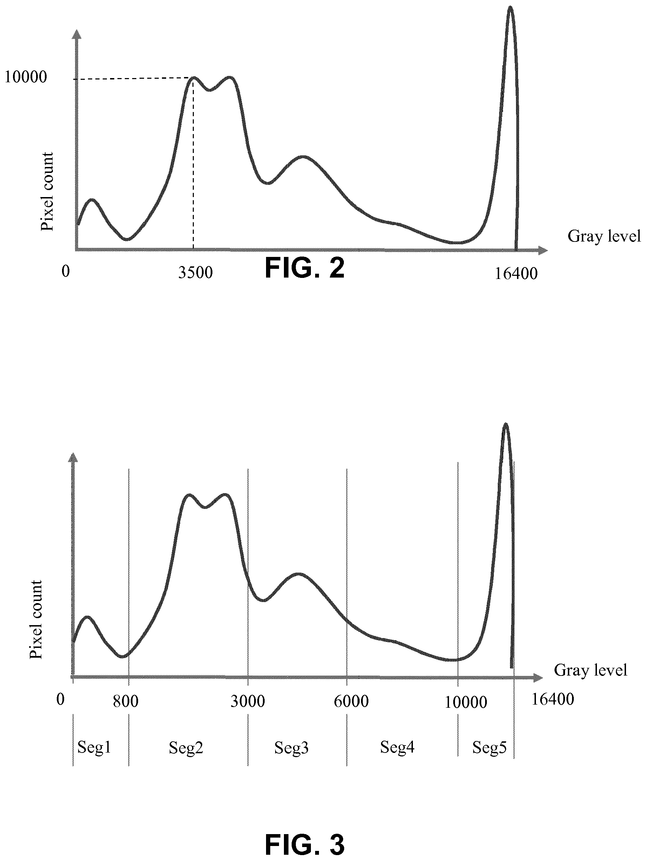

Figure 2 shows a histogram of grey levels of a typical panoramic radiographic image. The tissues crossed by X-rays attenuate X-rays in a way proportional to the atomic density of the tissue; as a consequence, given an input X-ray radiation, the denser tissue will absorb a higher quantity of radiation with respect to the less dense tissue; therefore in the first case a lesser quantity of X-ray radiation will impinge on the X-ray sensor with respect to less dense tissue. In practice, a black-and-white image will be obtained, ranging from pure white to pure black, passing through a gradation of intermediate greys. The number of intermediate grey levels depends on the quality of the acquisition system: typically today's digital acquisition system are designed with 16 bits and therefore can show 216 grey levels.

Any digital radiographic image consists of a pixel matrix, each of which has a gray level, determined by the quantity of X-rays attenuated by the passage through patient's tissues corresponding to said image pixel. Starting from grey levels, other kinds of more or less complex attenuation measure, such as e.g. Hounsfield Unit (HU), or conversion of grey scale to a polychromatic scale, can be derived: the method of the present invention can anyway be analogously applied to all cases in which the image is codified as a set of small units, each characterized by one or more values describing radiopacity. The man skilled in the art can use and combine the idea of this invention in any different way that technology and knowledge allow. In general, for each radiographic image a histogram like that of Figure 2 can be represented, bearing grey levels on X-axis and number of pixels having that grey level on Y-axis.

In the exemplary histogram shown in Figure 2, 10.000 pixels with a grey level of 3.500 are present. The histogram of the image is a way to show the quality of the radiographic image: if the pixels were present only in a fraction of grey levels, an image having insufficient contrast would result, wherein distinguishing among the different kinds of tissue would be difficult. Vice versa, a histogram showing a wide distribution of grey levels corresponds to a radiographic image wherein to the different kinds of tissue are assigned different grey levels, and therefore tissues are easily distinguishable from each other.

In Figure 3 a histogram prepared for use in the method of the present invention is shown. The grey levels (0 to 16.400) in the present example were subdivided into 5 sectors, each corresponding to the grey levels of a tissue typically represented in a panoramic image. E.g. the values attributable to each sector of Figure 3, assuming that the intensity of grey levels is a way to represent the radio-opacity of tissues, are

- Sector 1 [0-800]: metal and prosthesis
- Sector 2 [800-3000]: hard bone
- Sector 3 [3000-6000]: spongy bone
- Sector 4 [6000-10000]: soft tissues
- Sector 5 [10000-16400]: airways

The ends identifying a sector can be both fixed (static segmentation) and variable (dynamic segmentation). E.g. the segmentation can be correlated to particular trend lines of the histogram, or to minimum/maximum of a polynomial approximation of the histogram, etc.

The same sectors can have overlapping regions and not necessarily be adjoining to each other. The known types of mathematical/numerical analysis to get to histogram segmentation can be different and of different complexity. In the present embodiment a static segmentation in sectors without overlapping was chosen.

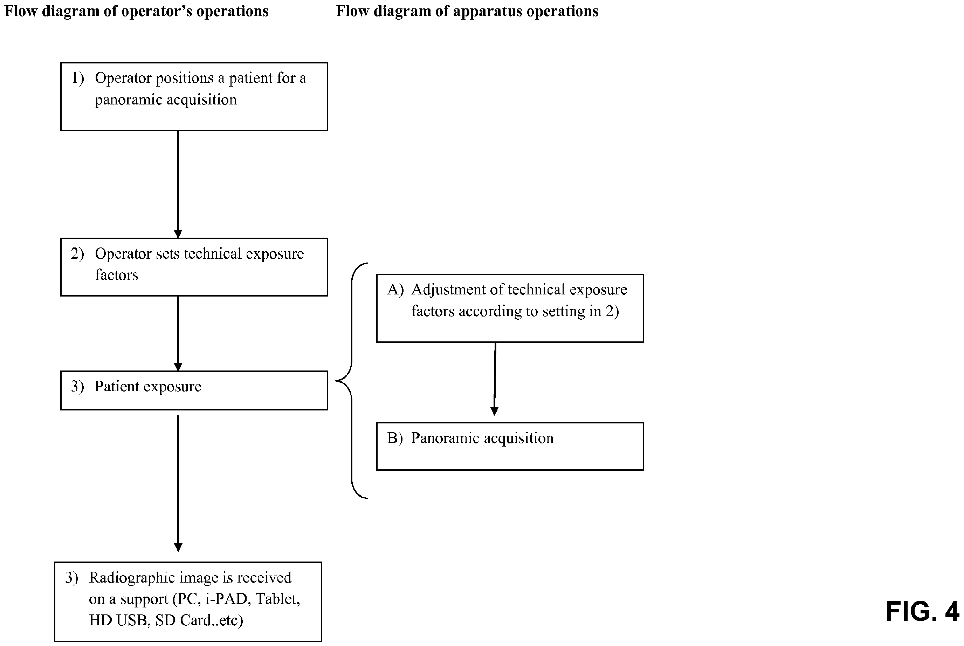

In Figure 4 the flow diagram of user's operation, i.e. the material actions that the operator must perform to acquire a panoramic image, and the flow diagram of the operations performed by the apparatus in the known art are shown.

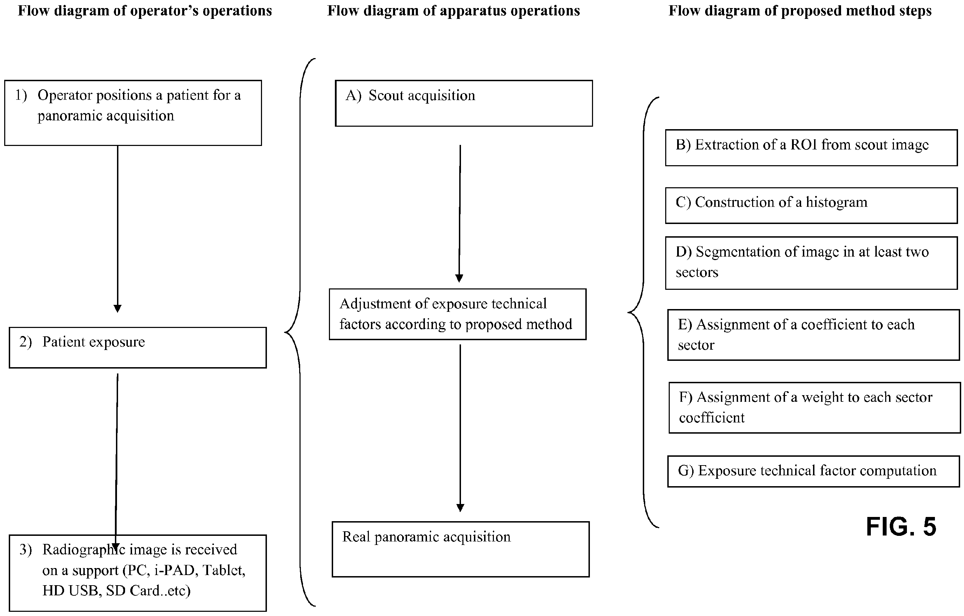

In Figure 5 the flow diagram of user's operation, the flow diagram of the operations performed by the apparatus, and the flow of the algorithm steps for computing the technical exposure factors according to the present invention are shown. Such diagram highlights, with respect to Figure 4:

- The absence of additional steps for operator/patient in the acquisition flow with respect to the known art;
- The integration of the method in the operative flow of the apparatus;
- The description of algorithm steps.

The method comprises the following steps:

A) Acquisition of a patient's scout image

The positioning of the patient is the standard positioning for a panoramic acquisition, so that the patient remains in the same position even during the real acquisition (meaning the acquisition of the panoramic image).

B) Extraction of a Region Of Interest (ROI) from the scout image

C) Construction of a histogram of grey levels of image pixels

similar to that of Figure 2.

D) Segmentation of the image according to a significant number of sectors

In the preferred embodiment shown in Figure 3, the number of sectors corresponds to five pre-set kinds of tissue, see above. Performing a segmentation according to different criteria is nonetheless possible. E.g. the histogram can be cut only for the values typical for metal, in order to study this portion of the image. Alternatively it is possible to detect the outline of the tissues having a very different radiopacity, so identifying the sectors of the histogram where a high gradient of grey levels is present.

E) Assignment of a coefficient to each sector

The coefficient is a number representative of its sector. The value of such coefficient can be pre-set or depending on the information present in scout acquisition. In the present case, referring to Figure 3 histogram, the coefficient of metal portion is a number given by the average of the values belonging to metal sector, and hard bone is the average of the values of hard bone sector; the same for the other sectors.

Example: Ki = arithmetic average of grey levels of the pixels belonging to each sector of interest. The values computed for each sector are:

K1:400 K2:2150 K3:5300 K4:8600 K5:15150

F) Assignment of a weight to each coefficient

The weight can be assigned to the coefficient by a human operator before performing the scout acquisition, or at the installation of the apparatus, according to diagnostic needs. In the preferred embodiment the coefficient weight is pre-set by the manufacturer, based on the fact that the kinds of tissue which can be found in a panoramic image are known. Always referring to Figure 3 as histogram example, we can set: W1 = W5 = 0, as optimizing the exposure for metal or airways is useless. Typically in a panoramic image hard bone is important, but not overexposing other tissues is also important.

In the preferred embodiment the definition of weights is based on how many pixels belong to each kind of tissue in the whole scout image, assuming that the scout acquisition is a representative sample of the group of tissues to be evaluated.

In the example of Figure 3, sectors 2, 3, 4 corresponding to hard bone, spongy bone and soft tissues will be considered only. For each sector weights W2, W3, W4 are computed, to be assigned to coefficient K2, K3, K4, comparing the number of pixels belonging to each sector and the total number of scout pixels.

For instance:

$$\texttt{For instance: Wi = Num(Segi)/Num(totROI)}$$

NB : Num(A) = number of pixels $\in$ A; totROI is the overall number of ROI (Region of Interest) pixels; Segi is the segment or sector of the histogram in this case sectors 2, 3 and 4.

As aa example of result for the sectors indicated above the following weights are computed.

$$W2 = 0.45 \quad W3 = 0.3 \quad W4 = 0.25$$

Obviously nothing prevents assigning weights according to other criteria, even independent from ROI informative content, but e.g. based on statistical or clinical elements (for edentulous patients assigning a higher weight to soft tissues might be advantageous, so as to make gum line more apparent).

G) Computation of technical exposure factors of the real acquisition

The computing of real exposure is a result of a function of product Ki*Wi of each sector, i.e. mathematically:

$$\texttt{[kV, mA, sec, filmspeed]=f(K1*W1, K2*W2, K3*W3, K4*W4, K5*W5)}$$

In the preferred embodiment, only kV are modulated with a weighted average of coefficients according to their weight. Defining more complex functions modifying the other technical exposure factors is nonetheless possible (mA, sec).

$$
f(x) = \begin{cases}
mA = 5 & \text{(fixed)} \\
sec = 9 & \text{(fixed)} \\
filmspeed = 10000 \text{ (fixed)} \\
kV = (a* K2*W2 + b* K3*W3 + c* K4*W4)/(K2*W2 + K3*W3 + K4*W4) \\
\quad = 74{,}7 \text{ kV}
\end{cases}
$$

In the example of Figure 3, a, b, c are values of kV suitably chosen based on the tissue to be irradiated. In a typical X ray tube in a panoramic apparatus, the value of kV can vary in a range of 60-80 kV.

The creation of the ROI histogram is a non-onerous operation, in that finishing the scout acquisition is not necessary to perform the extraction itself; the extraction can as a matter of fact occur while the sensor pixels are read. The method is based essentially on simple arithmetic calculation which can be performed by apparatus electronic system in the time interval between the end of scout acquisition and the beginning of the real panoramic acquisition, without the need of big memories or computing power, beyond those anyway necessary for the normal managing of panoramic images.

It is apparent that the present method of adjustment of technical exposure factors can be usefully used in any kind of radiologic apparatus.

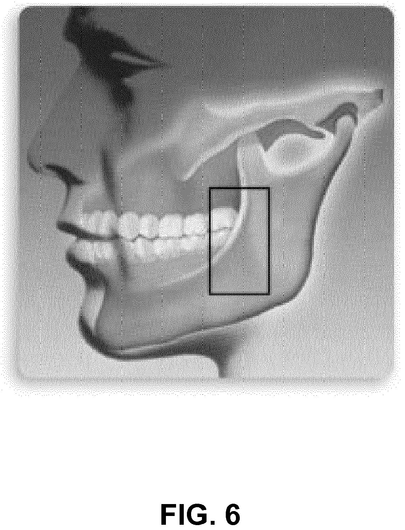

The preferred embodiment, as said above, relates to a panoramic radiographic apparatus. In this specific case, the area wherein the scout image is acquired is very important. The best results were obtained acquiring the scout image in the area highlighted by the square in Figure 6: an area comprising a portion of the ascending ramus of the mandible, including as well distal molars (seventh, eighth teeth), comprising as well a portion of the maxilla. In this way the three kinds of tissue (hard bone, spongy bone and soft tissues) are present in the scout and as a consequence in the histogram, and differentiating the histogram in the three meaningful sectors for the calculation of technical exposure factors is therefore possible.

An important advantage of the present invention consists in the fact that the calculation and the consequent adjustment of technical exposure factors occurs in a totally automatic way, without any operation by the operator, who must only suitably position the patient in the positioning device.

With respect to scout acquisition, some clarifications are needed. The scout exposes a limited portion of the patient: supposing that the time necessary for acquiring patient's whole dental arch is 100, the scout is performed exposing the patient for a time range of 1-8. All this occurs with the same technical exposure factors, therefore the X-ray dose administered to the patient for the scout is a small fraction of the total administered dose: this dose increase is justified by the fact that the total dose is then tailored on that specific patient, with a suitable cost/benefit ratio, in addition to preventing the risk of re-takes. In this way an X-ray dose with the technical exposure factors typical of the acquisition of real panoramic images is administered for a limited time: this is advantageous also for the whole acquisition time, which is only slightly prolonged.

In an alternative embodiment, administering an equivalent X-ray dose using a reduced current for a more prolonged time is possible, with equal X-ray dose.

In the preferred embodiment, scout acquisition can be considered as a fraction of the acquisition of the real panoramic image under every point of view: patient positioning, X-ray dose, time. It is nonetheless possible acquiring a scout image with specific collimation and trajectory.

Summing the fact that ROI extraction occurs at the same time as scout acquisition, and the fact that scout acquisition covers a small portion (1-8%) of the time of real panoramic acquisition, the acquisition of the real panoramic image is prolonged of a very small fraction of time. Moreover, the acquisition occurs positioning the patient in panoramic apparatus 1 through positioning devices 6, while scout and real panoramic acquisition occur one immediately after the other: this makes the acquisition comfortable both for patient and operator.

**Claims**

1. Method for patient specific, real time automatically adjusting at least one of technical exposure factors taken from the group consisting of:

   - X-ray tube power (kV),
   - X-ray tube current (mA),
   - Exposure duration (sec) and
   - Film speed

   for acquiring a radiographic image of a patient, the method comprising the following steps:

   A1) Positioning of a patient in an x ray imaging apparatus for acquisition of panoramic images, the said position remaining the same for the acquisitions of the panoramic images;
   A2) Performing a first acquisition of a scout image, in a specific anatomical area of the said patient using pre-defined parameters ;
   B) Extracting a Region Of Interest (ROI), from the said scout image;
   C) A grey level histogram of the image pixels of the ROI of the scout image is provided in which the grey levels are reported on an x-axis and the number of pixels having a certain one of the said grey levels on the y-axis; wherein
   D) The said histogram of the acquired image is subdivided into five sectors each corresponding to the grey level of a tissue typically represented in a panoramic image;
   E) At least a coefficient is assigned for each sector;
   F) A weight is assigned to each coefficient;
   G) At least an exposure parameter is computed according to the weights assigned to each sector;

   then the exposure of the real acquisition of a radiographic image is performed with the parameters computed according to the method.

2. Method according to claim 1, wherein said scout acquisition of step A) is performed in a fraction of time in a range of 1-8% with respect to total time needed for the real acquisition of the radiographic image, in a defined anatomical area containing tissues of clinical interest.

3. Method according to claim 2, wherein said scout acquisition is performed with the same values of X-ray tube power and film speed of the real acquisition for a limited time with respect to the real acquisition, using a higher current value.

4.  Method according to claim 1, wherein said segmentation of step C) can occur in a static or in a dynamic way.

5.  Method according to claim 1, wherein said coefficient of step D) is a function of average and standard deviation of pixels grey levels.

6.  Method according to claim 1, wherein for some sectors, not having a clinical interest, coefficient value is equal to zero.

7.  Method according to claim 1, wherein sector's coefficient weight is assigned according to the clinical importance of the tissue represented by the coefficient and/or to the kind of examination to be performed.

8.  Method according to any of the preceding claims, applied to the acquisition of a panoramic dental image.

9.  Method according to claim 8, wherein in step G) only X-ray power is adjusted.

10.  Apparatus (1) for panoramic radiography comprising an X-ray generator (2) adapted to project a collimated X-ray bundle through a patient, a bi-dimensional X-ray detector (3) adapted to be positioned so as to measure the intensity of radiation after it has crossed the patient, a C-arm (4) on which said X-ray source (2) and detector (3) are fixed on opposed ends, the position of the C-arm (4) being adjustable to the patient's height thanks to a vertically mobile post (7); a mechanical system (5) adapted to allow the rotation and translation of said support around the patient, to acquire radiographic images from different positions; a device (6) for positioning the patient; an electronic system adapted to control and synchronize the working of the various apparatus components, wherein the apparatus (1) is adapted to perform the method according to any of claims 1-9.

**Patentansprüche**

1.  Verfahren zur patientenspezifischen automatischen Echtzeit-Einstellung mindestens eines technischen Belichtungsfaktors aus der Gruppe bestehend aus:

    - Röntgenröhrenleistung (kV),
    - Röntgenröhrenstrom (rnA),
    - Belichtungsdauer (sec) und
    - Filmgeschwindigkeit

    zur Aufnahme eines Röntgenbildes eines Patienten, wobei das Verfahren die folgenden Schritte umfasst:

    A1) Positionieren eines Patienten in einer Röntgenbildgebungsvorrichtung zur Aufnahme von Panoramabildern, wobei die Position für die Aufnahmen der Panoramabilder gleich bleibt;
    A2) Durchführen einer ersten Aufnahme eines Scout-Bildes in einem bestimmten anatomischen Bereich des Patienten unter Verwendung von vordefinierten Parametern;
    B) Extrahieren eines interessierenden Bereichs (ROI) aus dem Scout-Bild;
    C) es wird ein Graustufenhistogramm der Bildpixel des ROI des Scout-Bildes bereitgestellt, bei dem die Graustufen auf einer x-Achse und die Anzahl der Pixel mit einer bestimmten Graustufe auf der y-Achse aufgetragen sind;
    wobei
    D) das Histogramm des aufgenommenen Bildes in fünf Sektoren unterteilt wird, die jeweils der Graustufe eines typischerweise in einem Panoramabild dargestellten Gewebes entsprechen;
    E) jedem Sektor wird mindestens ein Koeffizient zugeordnet;
    F) jedem Koeffizienten ist ein Gewicht zugeordnet;
    G) mindestens ein Belichtungsparameter in Abhängigkeit von den jedem Sektor zugeordneten Gewichten berechnet wird;

    anschließend erfolgt die Belichtung der realen Aufnahme eines Röntgenbildes mit den nach dem Verfahren berechneten Parametern.

2.  Verfahren nach Anspruch 1, wobei in einem definierten anatomischen Bereich, der Gewebe von klinischem Interesse enthält, die Scout-Aufnahme von Schritt a) in einem Bruchteil der Zeit in einem Bereich von 1-8% in Bezug auf die Gesamtzeit durchgeführt wird, die für die reale Aufnahme des Röntgenbildes benötigt wird.

3. Verfahren nach Anspruch 2, wobei die Scout-Aufnahme mit den gleichen Werten der Röntgenröhrenleistung und der Filmgeschwindigkeit der realen Aufnahme für eine begrenzte Zeit in Bezug auf die reale Aufnahme unter Verwendung eines höheren Stromwerts durchgeführt wird.

4. Verfahren nach Anspruch 1, wobei die Segmentierung von Schritt C) statisch oder dynamisch erfolgen kann.

5. Verfahren nach Anspruch 1, wobei der Koeffizient von Schritt D) eine Funktion der durchschnittlichen und standardmäßigen Abweichung der Pixel-Graustufen ist.

6. Verfahren nach Anspruch 1, wobei für einige Sektoren, die kein klinisches Interesse aufweisen, der Koeffizientenwert gleich Null ist.

7. Verfahren nach Anspruch 1, wobei das Koeffizientengewicht des Sektors in Abhängigkeit von der klinischen Bedeutung des durch den Koeffizienten repräsentierten Gewebes und/oder der Art der durchzuführenden Untersuchung zugeordnet wird.

8. Verfahren nach einem der vorhergehenden Ansprüche, angewendet auf die Aufnahme eines Panoramadentalbildes.

9. Verfahren nach Anspruch 8, wobei in Schritt G) nur die Röntgenleistung eingestellt wird.

10. Vorrichtung (1) für Panoramaradiographie umfassend einen Röntgengengenerator (2) zum Projizieren eines kollimierten Röntgenstrahlenbündels durch einen Patienten hindurch, einen zweidimensionalen Röntgendetektor (3) zum Messen der Intensität der Strahlung, nachdem sie den Patienten durchquert hat, einen C-Arm (4), an dem die Röntgenquelle (2) und Detektor (3) an gegenüberliegenden Enden befestigt sind, wobei die Position des C-Arms (4) durch eine vertikal bewegliche Säule (7) auf die Höhe des Patienten einstellbar ist; ein mechanisches System (5), das derart angepasst ist, um die Rotation und das Verfahren des Trägers um den Patienten herum zu ermöglichen und Röntgenbilder aus verschiedenen Positionen aufzunehmen; eine Vorrichtung (6) zum Positionieren des Patienten; ein elektronisches System, das dazu eingerichtet ist, die Arbeitsweise der verschiedenen Vorrichtungskomponenten zu steuern und zu synchronisieren, wobei die Vorrichtung (1) dazu eingerichtet ist, das Verfahren gemäß einem der Ansprüche 1-9 durchzuführen.

**Revendications**

1. Méthode spécifique par patient ajustant automatiquement en temps réel au moins un des facteurs techniques d'exposition compris parmi le groupe constitué:

   - de la puissance du tube à rayons X (kV),
   - du courant du tube à rayons X (mA),
   - de la durée d'exposition (sec.) et
   - de la vitesse du film

   pour acquérir une image radiographique d'un patient, la méthode comprenant les étapes suivantes:

   A1) Positionner un patient dans un appareil d'imagerie par rayons X pour l'acquisition d'images panoramiques, ladite position restant la même pour les acquisitions des images panoramiques;
   A2) Effectuer une première acquisition d'une image préliminaire ("scout"), dans une zone anatomique spécifique dudit patient à l'aide de paramètres prédéfinis;
   B) Extraire une Région d'Intérêt (ROI) de ladite image préliminaire;
   c) Un histogramme de niveaux de gris des pixels d'image de la région d'intérêt ROI de l'image préliminaire étant fourni et dans lequel les niveaux de gris sont reportés sur un axe des x et le nombre de pixels ayant un certain desdits niveaux de gris sur l'axe des y;
   dans laquelle
   D) Ledit histogramme de l'image acquise est subdivisé en cinq secteurs correspondant chacun au niveau de gris d'un tissu typiquement représenté dans une image panoramique;

E) Au moins un coefficient est attribué à chaque secteur;

F) Un poids est attribué à chaque coefficient;

G) Un moins un paramètre d'exposition est calculé en fonction des poids attribués à chaque secteur;

puis, l'exposition de l'acquisition réelle d'une image radiographique est effectuée avec les paramètres calculés selon la méthode.

2. Méthode selon la revendication 1, dans laquelle ladite acquisition préliminaire de l'étape A) est effectuée en une fraction de temps dans une plage de 1-8% par rapport au temps total nécessaire pour l'acquisition de l'image radiographique, dans une zone anatomique définie contenant des tissus d'intérêt clinique.

3. Méthode selon la revendication 2, dans laquelle ladite acquisition préliminaire est effectuée avec les mêmes valeurs de puissance du tube à rayons X et de vitesse du film de l'acquisition réelle pendant un temps limité par rapport à l'acquisition réelle, en utilisant une valeur de courant plus élevée.

4. Méthode selon la revendication 1, dans laquelle ladite segmentation de l'étape C) peut se produire de manière statique ou dynamique.

5. Méthode selon la revendication 1, dans laquelle ledit coefficient de l'étape D) est une fonction de la moyenne et de l'écart-type des niveaux de gris des pixels.

6. Méthode selon la revendication 1, dans laquelle pour certains secteurs n'ayant aucun intérêt clinique, la valeur du coefficient est égale à zéro.

7. Méthode selon la revendication 1, dans laquelle le poids du coefficient du secteur est attribué en fonction de l'importance clinique du tissu représenté par le coefficient et/ou en fonction du type d'examen à effectuer.

8. Méthode selon l'une quelconque des revendications précédentes, appliquée à l'acquisition d'une image dentaire panoramique.

9. Méthode selon la revendication 8, dans laquelle dans l'étape G) seule la puissance des rayons X est ajustée.

10. Appareil (1) pour une radiographie panoramique comprenant un générateur de rayons X (2) adapté pour projeter un faisceau de rayons X collimaté au travers d'un patient, un détecteur de rayons X bidimensionnel (3) adapté pour être positionné de façon à mesurer l'intensité de la radiation après avoir traversé le patient, un bras en forme de C (4) sur lequel ladite source de rayons X (2) et le détecteur (3) sont fixés à des extrémités opposées, la position du bras en forme de C (4) pouvant être ajustée à la hauteur du patient grâce à une colonne verticalement mobile (7); un système mécanique (5) adapté pour permettre la rotation et le translation dudit support autour du patient, afin d'acquérir des images radiographiques à partir de différentes positions; un dispositif (6) de positionnement du patient; un système électronique adapté pour contrôler et synchroniser le fonctionnement des divers composants de l'appareil, dans lequel l'appareil (1) est adapté pour effectuer la méthode selon quelconque des revendications 1-9.

**FIG. 1**

FIG. 2

FIG. 3

**Flow diagram of operator's operations**     **Flow diagram of apparatus operations**

1) Operator positions a patient for a panoramic acquisition

2) Operator sets technical exposure factors

A) Adjustment of technical exposure factors according to setting in 2)

3) Patient exposure

B) Panoramic acquisition

3) Radiographic image is received on a support (PC, i-PAD, Tablet, HD USB, SD Card..etc)

**FIG. 4**

**Flow diagram of operator's operations**   **Flow diagram of apparatus operations**   **Flow diagram of proposed method steps**

1) Operator positions a patient for a panoramic acquisition

A) Scout acquisition

B) Extraction of a ROI from scout image

C) Construction of a histogram

D) Segmentation of image in at least two sectors

2) Patient exposure

Adjustment of exposure technical factors according to proposed method

E) Assignment of a coefficient to each sector

F) Assignment of a weight to each sector coefficient

G) Exposure technical factor computation

3) Radiographic image is received on a support (PC, i-PAD, Tablet, HD USB, SD Card..etc)

Real panoramic acquisition

**FIG. 5**

**FIG. 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4618974 A
- EP 2574280 A
- US 4813060 A
- US 5425065 A
- EP 0574368 A
- US 5124913 A
- US 4333012 A, Morita
- EP 1161122 A, Palodex